(19) Europäisches Patentamt
European Patent Office
Office européen des brevets

(11) Veröffentlichungsnummer: **0 054 227**
**B1**

# (12) EUROPÄISCHE PATENTSCHRIFT

(45) Veröffentlichungstag der Patentschrift: **28.09.83**

(51) Int. Cl.[3]: **C 07 C 45/58**, C 07 C 49/167, C 07 C 37/20

(21) Anmeldenummer: **81110102.1**

(22) Anmeldetag: **03.12.81**

(54) **Verfahren zur Herstellung von Hexafluoraceton sowie Verwendung der nach dem Verfahren zunächst erhaltenen Lösung.**

(30) Priorität: **11.12.80 DE 3046604**

(43) Veröffentlichungstag der Anmeldung: **23.06.82 Patentblatt 82/25**

(45) Bekanntmachung des Hinweises auf die Patenterteilung: **28.09.83 Patentblatt 83/39**

(84) Benannte Vertragsstaaten: **BE CH DE FR GB IT LI NL**

(56) Entgegenhaltungen:
**US-A-3 321 515**
**US-A-3 385 904**
**CHEMICAL ABSTRACTS, Band 67, Nr. 23, 1967, Seite 10180, Nr. 108144x Columbus, Ohio, U.S.A. K. WIECHERT et al.: »Isomerization of oxiranes to aldehydes and ketones by anhydrous, liquid hydrogen fluoride in the presence of acetonitrile«**

(73) Patentinhaber: **HOECHST AKTIENGESELLSCHAFT, Postfach 80 03 20, D-6230 Frankfurt am Main 80 (DE)**

(72) Erfinder: **Rammelt, Peter-Paul, Dr., Ublerstrasse 33, D-6238 Hofheim am Taunus (DE)**
Erfinder: **Siegemund, Günter, Dr., Frankfurter Strasse 21, D-6238 Hofheim am Taunus (DE)**

EP 0 054 227 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von Hexafluoraceton sowie Verwendung der nach dem Verfahren zunächst erhaltenen Lösung

Hexafluoraceton $CF_3COCF_3$ ist ein wertvolles Zwischen- und Endprodukt auf verschiedenen Sachgebieten. So ist Hexafluoraceton als Zwischenprodukt von Bedeutung z. B. bei der Copolymerisation mit Perfluorolefinen, zur Herstellung von Pflanzenschutzmitteln oder von Vernetzungsmitteln für fluorhaltige Elastomere. Derartige Vernetzungsmittel sind z. B. die nach I. L. Knunyants et al. [Isz. Akad. Nauk SSSR, Otdel. Khim. Nauk 4, 686 bis 692 (1960) — englische Ausgabe S. 647—653, insbesondere 649 — 650] durch Umsetzung von Hexafluoraceton mit Phenol oder o-Kresol in wasserfreiem Fluorwasserstoff erhältlichen Diphenole 4,4'-(Hexafluorisopropyliden)diphenol = Hexafluor-2.2-bis-(4-hydroxyphenyl)-propan (I) und 4,4'-(Hexafluorisopropyliden)-di-o-Kresol = Hexafluor-2.2-bis-(3-methyl-4-hydroxyphenyl)-propan (II):

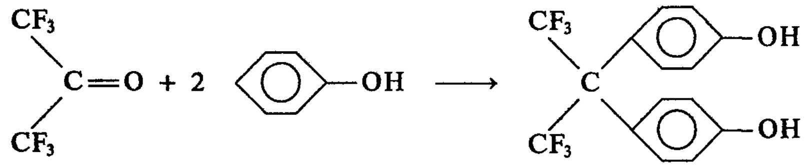
(I)

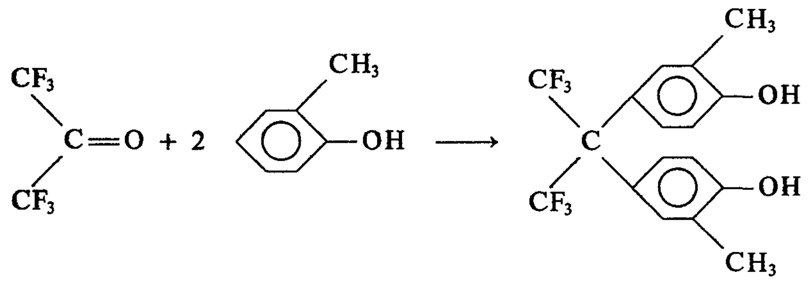
(II)

Als Endprodukt spielt Hexafluoraceton eine nicht unwichtige Rolle u. a. als Lösungsmittel für fluorhaltige Polymere etc.

Die Herstellung des Hexafluoracetons erfolgt zweckmäßig durch Umlagerung (Isomerisierung) von Hexafluorpropenepoxid, das seinerseits vorteilhaft z. B. durch anodische Oxidation von Hexafluorpropen nach dem in der DE-PS 24 60 468 und deren Zusatz 26 58 328 beschriebenen Verfahren in der Elektrolysezelle gemäß DE-PS 26 58 382 erhalten werden kann.

Es ist bekannt, u. a. die Umlagerung von Hexafluorpropenepoxid zu Hexafluoraceton in Gegenwart von Antimonpentafluorid $SbF_5$ bei Temperaturen zwischen etwa $-15$ und $+350°C$ durchzuführen (US-PS 3 213 134); das einzige auf diese Umlagerung gerichtete Beispiel (Nr. 5) enthält allerdings keinerlei Ausbeuteangaben. In dieser US-PS wird auch besonders darauf hingewiesen, daß die Umlagerung in Abwesenheit von Fluorwasserstoff durchzuführen ist, da anderenfalls — also in Gegenwart von Fluorwasserstoff — hauptsächlich der entsprechende Alkohol (durch Fluorwasserstoffanlagerung) entstehen soll.

Die Alkoholbildung infolge der Anlagerung von Fluorwasserstoff an perfluorierte Epoxide wird ausgenutzt bei dem Verfahren zur Herstellung perfluorierter tertiärer Alkohole durch Reaktion von Perfluorisoalken-1.2-epoxiden von mindestens 4 und nicht mehr als 20 C-Atomen mit wasserfreiem Fluorwasserstoff (gegebenenfalls in Gegenwart von Katalysatoren wie $SbF_5$, CsF etc.) bei Temperaturen zwischen etwa $+20$ und $+350°C$ gemäß US-PS 3 385 904. Dieses Verfahren läßt sich durch folgende Reaktionsgleichung wiedergeben:

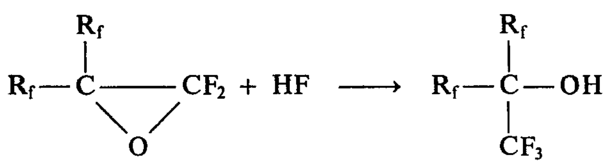

($R_f$ = Perfluoralkyl)

Eine weitere bekannte Methode der Umlagerung von Hexafluorpropenepoxid zu Hexafluoraceton arbeitet mit sog. Lewis-Säuren (d. s. Elektronenakzeptor-Verbindungen) als Katalysatoren (US-PS 3 321 515). Als hier infragekommende Lewis-Säuren sind bestimmte saure Metalloxide, Metallhalogenide und -carbonyle genannt. In den sich auf die Umlagerung von Hexafluorpropenoxid zu Hexafluoraceton beziehenden Beispielen (Nr. 1, 3 und 4) werden mit saurem Aluminiumoxid bzw. Aluminiumchlorid als Katalysatoren Ausbeuten an Hexafluoraceton zwischen etwa 50 und 80% d. Th. erreicht.

Mit sog. Lewis-Basen (d. s. Elektronendonator-Verbindungen) als Katalysatoren wird die Umlagerung des Hexafluorpropenepoxids in eine andere Richtung, nämlich auf die Bildung des entsprechenden Säurefluorids hin gelenkt. Das nachstehende Reaktionsschema veranschaulicht die beiden Umlagerungsmöglichkeiten des Hexafluorpropenepoxids, je nachdem ob eine Lewis-Säure oder eine Lewis-Base als Katalysator verwendet wird:

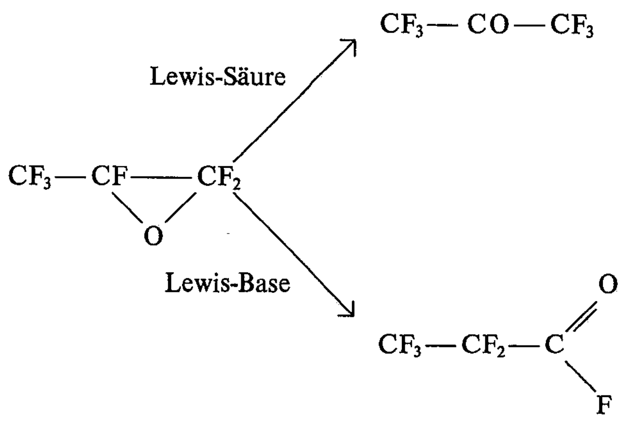


Als Lewis-Basen werden u. a. zur Abspaltung von Fluorionen befähigte Fluorverbindungen wie z. B. KF; $KHF_2$ etc. aufgeführt. Dabei sollen für die katalytische Aktivität der Verbindungen als Lewis-Basen die Fluorionen verantwortlich sein. Auch zur Abspaltung von Fluorionen befähigte, sonst als Lewis-Säuren anzusehende Verbindungen sollen wegen der abspaltbaren Fluorionen bei der fraglichen Umlagerung als Lewis-Basen wirken.

Das nach den bekannten Verfahren durch Umlagerung von Hexafluorpropenepoxid in Gegenwart von Katalysatoren hergestellte Hexafluoraceton enthält natürlich zunächst noch die eingesetzten Katalysatoren, was für verschiedene Verwendungszwecke des Hexafluoracetons störend ist. Deswegen ist in diesen Fällen eine Abtrennung des Katalysators erforderlich. Eine solche Katalysator-Abtrennung ist jedoch — insbesondere wenn die Katalysatoren in gelöster Form vorliegen — nicht immer ganz einfach und häufig sogar nur mit recht erheblichem Aufwand (wenn überhaupt) möglich.

Es bestand daher die Aufgabe, die bekannten Verfahren zur Herstellung von Hexafluoraceton so zu verbessern, daß das Reaktionsprodukt keinen bei der Weiterverwendung des Hexafluoracetons störenden Katalysator mehr enthält.

In Chem. Abstracts, Vol. 67, Nr. 23 (1967), Referat Nr. 108 144x ist zwar die Umlagerung fluorfreier sauerstoffhaltiger organischer 3-Ring-Verbindungen (Oxirane) mit Fluorwasserstoff HF in Acetonitril $CH_3CN$ — also bei verschiedenen weiteren Reaktionen nicht störenden und gegebenenfalls auch leicht entfernbaren und deswegen später nicht mehr störenden Verbindungen — zu den entsprechenden Aldehyden und Ketonen beschrieben:

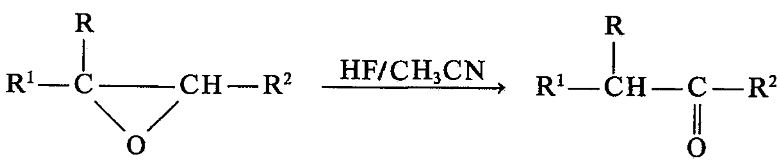


R, $R^1$, $R^2 = C_1-C_4$-Alkylgruppen oder Wasserstoff, wobei mindestens einer dieser Reste R, $R^1$ und $R^2$ eine der genannten Alkylgruppen ist, also nicht alle drei Reste gleich Wasserstoff sein können.

In der diesem Referat zugrundeliegenden Originalarbeit Z. Chem. 7(6), 229—230 (1967) heißt es jedoch, daß Monoalkyloxirane unter diesen Bedingungen nur Polymerisate bilden. Ein Monoalkyloxiran ist z. B. das fluorfreie Analogon des Hexafluorpropenepoxids, d. i. das (normale) Propenepoxid:

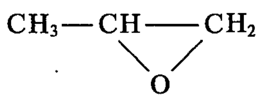

Dieses liefert also nach der vorerwähnten Literatur mit $HF/CH_3CN$ nicht etwa Aceton, sondern Polymerisate.

Es war daher außerordentlich überraschend als gefunden wurde, daß sich Hexafluorpropenepoxid mit Fluorwasserstoff als Katalysator und gegebenenfalls Lösungsmittel glatt in Hexafluoraceton umlagern läßt. Da Fluorwasserstoff keinen bei der Weiterverwendung des Hexafluoracetons störenden Katalysator darstellt, konnte damit auch die Aufgabe, die bekannten Verfahren zur Herstellung von Hexafluoraceton so zu verbessern, daß das Reaktionsprodukt keinen bei der

Weiterverwendung des Hexafluoracetons störenden Katalysator mehr enthält, gelöst werden.

Erfindungsgegenstand ist daher ein Verfahren zur Herstellung von Hexafluoraceton durch Umlagerung von Hexafluorpropenepoxid bei erhöhter Temperatur in Gegenwart eines sauren Katalysators; das Verfahren ist dadurch gekennzeichnet, daß als saurer Katalysator und gegebenenfalls als Lösungsmittel Fluorwasserstoff verwendet wird.

Durch diese Verfahrensweise ist es möglich, bei hohen (nahezu quantitativen) Umsätzen und einer hohen Selektivität Ausbeuten an Hexafluoraceton von über 90% d. Th. zu erreichen. Dies war in keiner Weise zu erwarten, weil man nach dem einschlägigen Stand der Technik annehmen mußte, daß Hexafluorpropenepoxid in Gegenwart von Fluorwasserstoff teils (unter Fluorwasserstoffanlagerung) den entsprechenden perfluorierten Alkohol (wegen US-PS 3 312 134 und US-PS 3 385 904), teils das entsprechende Säurefluorid (wegen US-PS-3 321 515; Lenkung der Umlagerung in Richtung auf das Säurefluorid durch Fluorionen !) oder eventuell nur Polymerisate (wegen Z. Chem. 7[6], 229—30 [1967]), ergibt.

Bei der Durchführung des erfindungsgemäßen Verfahrens ist zur Gewährleistung eines hinreichenden Umsatzes der Fluorwasserstoff zweckmäßig nicht nur in einer katalytischen Menge sondern im Überschuß — also auch als Lösungsmittel — einzusetzen. Vorzugsweise beträgt das molare Verhältnis von Hexafluorpropenepoxid zu Fluorwasserstoff 1 zu mindestens etwa 4, vorzugsweise 1 zu etwa 7 bis 9. Ein größerer Fluorwasserstoffüberschuß ist an sich nicht schädlich, aber aus Gründen der aufwendigeren Aufarbeitung auch nicht von Vorteil.

Die Reaktionstemperatur sollte zur Gewährleistung einer hinreichenden Reaktionsgeschwindigkeit und eines hinreichenden Umsatzes oberhalb etwa 65° C liegen; sie liegt vorzugsweise zwischen etwa 70 und 150° C, insbesondere zwischen etwa 90 und 125° C. Höhere Temperaturen sind an sich möglich, führen aber zur Bildung von Nebenprodukten wie z. B. Trifluoracetylfluorid und Trifluormethan, und infolgedessen zur Verringerung der Ausbeute an Hexafluoraceton.

Der Reaktionsdruck ist an sich nicht kritisch. Er ist abhängig von der Füllmenge der Reaktanten Hexafluorpropenepoxid und Fluorwasserstoff, bezogen auf den Inhalt des Reaktors, dem Gewichtsverhältnis zwischen Hexafluorpropenepoxid und Fluorwasserstoff und der angewandten Reaktionstemperatur. Er beträgt im allgemeinen zu Beginn der Reaktion zwischen etwa 30 und 50 bar und fällt am Reaktionsende normalerweise auf etwa 20 bar und darunter.

Das Verfahren kann im Autoklaven ohne oder mit einer Rührvorrichtung durchgeführt werden. Die für die Durchführung der Reaktion benötigte Apparatur kann aus allen hinreichend korrosionsbeständigen Werkstoffen bestehen wie z. B. Eisen, Chrom, Nickel und Edelmetallen sowie deren Legierungen.

Die Reaktionszeit beträgt zur Gewährleistung eines hinreichenden Umsatzes in dem angegebenen Temperaturbereich im allgemeinen zwischen etwa 10 und 25 Stunden, vorzugsweise zwischen etwa 20 und 24 Stunden.

Das erfindungsgemäße Verfahren wird im allgemeinen so durchgeführt, daß man Hexafluorpropenepoxid und Fluorwasserstoff in einen Autoklaven füllt und das Gemisch bis zur Beendigung der Umsetzung auf die Reaktionstemperatur erwärmt. Der sich infolge des Dampfdruckes des Gemisches ausbildende Druck nimmt mit fortschreitender Umsetzung ständig ab (wahrscheinlich wegen der Bildung eines lockeren Additionsprodukts aus HF und dem Hexafluoraceton) und kann als ungefährer Anhalt für das Reaktionsende benutzt werden. Nach Abschluß der Reaktion liegt das Hexafluoraceton gelöst in Fluorwasserstoff vor und kann entweder nach Abtrennung des Fluorwasserstoffs (z. B. durch Waschen mit Oleum) oder in der in Fluorwasserstoff gelösten Form weiter verwendet werden. Da zahlreiche Reaktionen mit Hexafluoraceton in Fluorwasserstoff als Lösungsmittel durchgeführt werden, kann in solchen Fällen die nach dem erfindungsgemäßen Verfahren erhaltene Reaktionslösung vorteilhaft gleich als solche ohne gesonderte Isolierung des Hexafluoracetons für die entsprechenden weiteren Umsetzungen eingesetzt werden.

Gegenüber den vergleichbaren Verfahren des einschlägigen Standes der Technik besitzt das erfindungsgemäße Verfahren — insbesondere dann, wenn die Reaktionslösung gleich für weitere Umsetzungen des Hexafluoracetons in Fluorwasserstoff verwendet wird — den Vorteil, daß keine bei den weiteren Umsetzungen gegebenenfalls störenden Katalysatoren entfernt werden müssen. Außerdem werden bei dem Verfahren durchweg höhere Ausbeuten wie nach den bekannten Verfahren erhalten.

Es ist besonders bevorzugt, die nach dem erfindungsgemäßen Verfahren erhaltene Reaktionslösung direkt — d. h. ohne irgendeine Aufarbeitung oder Reinigung — für die weitere Umsetzung mit Phenol oder o-Kresol nach Knunyants et al. a.a.O. zu verwenden. Dabei kann man beispielsweise so vorgehen, daß man Hexafluorpropenepoxid bei etwa 100° C in der etwa 8fachen molaren Menge Fluorwasserstoff in einem Druckgefäß umlagert, anschließend die erforderliche Menge Phenol oder o-Kresol zufügt und die Reaktion zum Hexafluor-2.2-bis-(4-hydroxyphenyl)-propan bzw. Hexafluor-2.2-bis-(3-methyl-4-hydroxyphenyl)-propan bei der gleichen Temperatur durchführt. Zur Aufarbeitung wird der Fluorwasserstoff abdestilliert und kann erneut zu weiteren Umsetzungen eingesetzt werden. Das Reaktionsprodukt Hexafluor-2.2-bis-(4-hydroxyphenyl)-propan bzw. Hexafluor-2.2-bis-(3-methyl-4-hydroxyphenyl)-propan bleibt als feste Substanz zurück und kann in üblicher Weise gereinigt werden.

Die Erfindung wird nun durch die nachstehenden Beispiele näher erläutert.

## Beispiel 1 bis 6

Diese Beispiele erläutern den Effekt der Temperatur, des Molverhältnisses von Hexafluorpropenepoxid zu Fluorwasserstoff und der Reaktionszeit auf die Umlagerung des Hexafluorpropenepoxids.

Die Umlagerung wurde in einem Stahlautoklaven von 250 ml Inhalt durchgeführt, der mit Hexafluorpropenepoxid, das ca. 0,3 Mol-% Hexafluorpropen und bis zu 4 Mol-% cyclo-Hexafluorpropan enthielt, und Fluorwasserstoff gefüllt wurde. Die Umlagerung wurde durch Erwärmen des Reaktionsgefäßes auf die gewünschte Reaktionstemperatur unter Schütteln in Gang gesetzt. Nach Ablauf der gewünschten Reaktionszeit wurde das Reaktionsprodukt auf 40—50° C abgekühlt und zur Befreiung von Fluorwasserstoff mit 65%igem Oleum gewaschen. Die Zusammensetzung des Reaktionsproduktes nach der Oleum-Wäsche ist in Tabelle 1 dargestellt.

Tabelle 1

| Bsp. | Hexafluor-propenepoxid g | Fluor-wasserstoff g | Mol-verhältnis HF : HFPO*) | Reakt.-Temp. °C | Reakt.-Zeit Std. |
|---|---|---|---|---|---|
| 1 | 110 | 70 | 5.3 | 85 | 5 |
| 2 | 100 | 50 | 4.2 | 100 | 19 |
| 3 | 100 | 110 | 9.1 | 100 | 10 |
| 4 | 100 | 110 | 9.1 | 100 | 15 |
| 5 | 100 | 100 | 8.3 | 100 | 19 |
| 6 | 110 | 100 | 7.6 | 150 | 19 |

Tabelle 1 (Fortsetzung)

| Bsp. | Zusammensetzung des Reaktionsproduktes in Mol.-% | | | | | | | | |
|---|---|---|---|---|---|---|---|---|---|
| | Hexa-fluor-aceton | HFPO*) | $CF_3$ \| COF | $CHF_3$ | $C_3F_6$ | $C_3F_6$ cycl. | unbek. Komp. | Umsatz % | Selektivität % |
| 1 | 2.6 | 95.7 | <0.3 | 0.1 | <0.3 | 1.7 | 0.1 | 2.7 | 96.3 |
| 2 | 61.7 | 33 | 0.6 | 0.3 | <0.3 | 3.3 | 1.1 | 65.9 | 96.9 |
| 3 | 88 | 7.6 | 0.8 | 0.2 | 0.2 | 1.1 | 2.2 | 92.3 | 96.5 |
| 4 | 92.4 | 4.2 | <0.3 | 0.2 | <0.3 | 1.8 | 1.1 | 95.7 | 98.3 |
| 5 | 92.5 | 0.9 | 0.3 | 0.1 | 0.2 | 0.8 | 5.5 | 99.1 | 94.3 |
| 6 | 68.2 | 2.4 | 17.8 | 4.7 | 0.5 | 2.4 | 4 | 97.5 | 72.0 |

*) HFPO = Hexafluorpropenepoxid

## Beispiel 7

In einen Stahlautoklaven von 250 ml Inhalt wurden 100 g Hexafluorpropenepoxid, das ca. 0.3 Mol-% Hexafluorpropen und ca. 0.6 Mol-% cyclo-Hexafluorpropan enthielt, und 100 g Fluorwasserstoff gefüllt und 24 Stunden unter Schütteln auf 100°C erhitzt. Das auf 40—50°C abgekühlte Reaktionsprodukt wurde zur Befreiung von Fluorwasserstoff mit 65%igem Oleum gewaschen und in einer auf −78°C gekühlten Vorlage 79 g kondensiert. Das Reaktionsprodukt hatte folgende Zusammensetzung (in Mol-%):

| Hexa-fluor-aceton | Hexa-fluor-propen-epoxid | $CF_3-COF$ | $CHF_3$ | $C_3F_6$ | cyclo-$C_3F_6$ | unbek. Komp. | Umsatz % | Selek-tivität % |
|---|---|---|---|---|---|---|---|---|
| 95.3 | 1.1 | <0.2 | <0.3 | <0.3 | 2.2 | 1.1 | 98.9 | 98.9 |

Beispiel 8

In einen Autoklaven aus rostfreiem Stahl von 1 Liter Inhalt ohne Rührvorrichtung wurde ein Gemisch aus 228 g Hexafluorpropenepoxid und 72 g Hexafluorpropen, sowie 220 g Fluorwasserstoff gefüllt und 20 Stunden auf 100°C erhitzt. Das Reaktionsprodukt, das aus einer Lösung von Hexafluoraceton in Fluorwasserstoff bestand, wurde unter Ausnutzung seines eigenen Dampfdruckes in einen Nickelautoklaven von 2.7 Liter Inhalt umgefüllt, in dem 258 g Phenol vorgelegt waren und 8 Stunden auf 100°C erhitzt. Nach dem Abdestillieren des Fluorwasserstoffs und des Hexafluorpropens, das sich nicht an der Reaktion beteiligt, wurden 399 g Hexafluor-2.2-bis-(4-hydroxyphenyl)-propan erhalten. Das entspricht einer Ausbeute von 86.4% d. Th.

**Patentansprüche**

1. Verfahren zur Herstellung von Hexafluoraceton durch Umlagerung von Hexafluorpropenepoxid bei einer Temperatur zwischen etwa 60 und 150°C, vorzugsweise zwischen etwa 90 und 125°C, in Gegenwart eines sauren Katalysators, dadurch gekennzeichnet, daß als saurer Katalysator und gegebenenfalls als Lösungsmittel Fluorwasserstoff verwendet wird.

2. Verfahren nach Anspruch 1, dadurch gekennzeichnet, daß man die Umlagerung bei einem molaren Verhältnis von Hexafluorpropenepoxid zu Fluorwasserstoff von 1 zu mindestens etwa 4, vorzugsweise von 1 zu etwa 7 bis 9 durchführt.

3. Verwendung der nach dem Verfahren gemäß den Ansprüchen 1 bis 3 erhaltenen Lösung von Hexafluoraceton in Fluorwasserstoff zu direkten weiteren Umsetzungen, vorzugsweise mit Phenol und/oder o-Kresol.

**Claims**

1. A process for the preparation of hexafluoroacetone by a rearrangement of hexafluoropropene epoxide at a temperature between about 60 and 150°C, preferably between about 90 to 125°C, in the presence of an acid catalyst, characterized in using as acid catalyst and optionally as solvent hydrogen fluoride.

2. A process as claimed in claim 1, characterized in carrying out the rearrangement at a molar ratio of hexafluoropropene epoxide to hydrogen fluoride of 1 to at least about 4, preferably of 1 to about 7 to 9.

3. The use of the solution of hexafluoroacetone in hydrogen fluoride, which is obtained by the process in accordance with claims 1 to 2, in direct further reactions, preferably with phenol and/or o-cresol.

**Revendications**

1. Procédé de préparation d'hexafluoroacétone par transposition d'époxyde d'hexafluoropropène à une température d'environ 60 à 150°C, de préférence d'environ 90 à 125°C, en présence d'un catalyseur acide, procédé caractérisé en ce que l'on utilise du fluorure d'hydrogène comme catalyseur acide, et éventuellement aussi comme solvant.

2. Procédé selon la revendication 1, caractérisé en ce que la transposition est effectuée avec un rapport molaire de l'époxyde d'hexafluoropropène au fluorure d'hydrogène de 1 à au moins 4 environ, de préférence de 1 à environ 7 à 9.

3. L'utilisation directe de la solution d'hexafluoroacétone dans le fluorure d'hydrogène, obtenue par le procédé selon la revendication 1 ou 2, pour d'autres réactions ultérieures préférence avec le phénol et/ou l'ortho-crésol.